Europäisches Patentamt

European Patent Office

Office européen des brevets

(11) Publication number: **0 203 785**
**B1**

(12) EUROPEAN PATENT SPECIFICATION

(45) Date of publication of patent specification: **19.09.90**

(51) Int. Cl.⁵: **A 61 F 2/18**

(21) Application number: **86303904.6**

(22) Date of filing: **22.05.86**

(54) Middle ear prosthesis.

(30) Priority: **24.05.85 US 737426**

(43) Date of publication of application:
**03.12.86 Bulletin 86/49**

(45) Publication of the grant of the patent:
**19.09.90 Bulletin 90/38**

(84) Designated Contracting States:
**BE CH DE FR GB IT LI**

(56) References cited:
**AT-B- 282 052**
**DE-A-3 036 245**
**DE-A-3 211 209**
**DE-B-2 905 183**
**US-A-3 909 852**
**US-A-4 510 627**

(73) Proprietor: **UNIVERSITY OF FLORIDA**
**223 Grinter Hall**
**Gainesville Florida 32611 (US)**

(72) Inventor: **Merwin, Gerald E.**
**3945 Northwest 35th Place**
**Gainesville, Florida 32606 (US)**
Inventor: **Spilman, Derek B.**
**4708 Southwest 67th Terrace**
**Gainesville, Florida 32608 (US)**
Inventor: **Hench, Larry L.**
**1415 Northwest 94th Street**
**Gainesville, Florida 32606 (US)**

(74) Representative: **Hedley, Nicholas James**
**Matthew et al**
**TZ Gold & Company 9 Staple Inn**
**London WC1V 7QH (GB)**

## Description

Background of invention
Field of invention

The present invention relates to a novel middle ear prosthesis.

Prior art

DE—A—3 036 245 describes an auditory prosthesis comprising a base plate and a shaft projecting from the plate either perpendicularly or at an angle; grooves are cut on the surface of the plate remote from the shaft. The prosthesis is made of bioactive composite material, e.g. methylmethacrylate with dispersions of a bioactive glass.

DE—A—3 211 209 describes an auditory prosthesis consisting of a part made of bio-active material and a part consisting of a bio-inert material. The prosthesis has a base plate with grooves on one face and a shaft extending from the other face at an angle to the plate.

Summary of the invention

The present invention comprises middle ear prostheses constructed of a bio-active glass having specific shapes and designs especially adapted to meet specific surgical needs in the reconstruction of all or part of the ossicular chain. The bioactive glass has the following weight composition:

| Component | Weight percentage |
|-----------|-------------------|
| $SiO_2$ | 40—52 |
| CaO | 20—50 |
| $Na_2O$ | 10—35 |
| $P_2O_5$ | 2—8 |
| $CaF_2$ | 0—25 |
| $B_2O_3$ | 0—10 |

One embodiment of the invention comprises a middle ear prosthesis comprising a substantially disc-like base member, one base surface of which is especially adapted to interface with and bond to the thin collagen layer of the tympanic membrane or grafted tympanic membrane of the middle ear and an integral substantially cylindrical stem member projecting outwardly from the other base surface of the base member, the stem member having a smaller diameter than the base member and the axis of the stem member being off-set with respect to the center of and substantially perpendicular to the base surface of the base member.

Another embodiment of the invention comprises a middle ear prosthesis comprising a substantially disc-like base member, one base surface of which is especially adapted to interface with and bond to the thin collagen layer of the tympanic membrane or grafted tympanic membrane of the middle ear and an integral substantially cylindrical stem member projecting outwardly from the other base surface of the base member, the stem member having a smaller diameter than the base member and the axis of the stem member being off-set with respect to the center of and substantially non-perpendicular to the base surface of the base member.

An additional embodiment of the invention comprises a middle ear prosthesis comprising a base member having two opposed flat surfaces, one of the flat surfaces being especially adapted to interface with and bond to a malleus handle of the middle ear and an integral substantially cylindrical stem member projecting outwardly from the other of the flat surfaces of the base member, the stem member having a smaller cross-section than the base member and the axis of the stem member being off-set with respect to the center of and substantially perpendicular to the base surface of the base member.

A further embodiment of the invention comprises a middle ear prosthesis comprising a base member having two opposed flat surfaces, one of the flat surfaces being especially adapted to interface with end bond to a malleus handle of the middle ear and an integral substantially cylindrical stem member projecting outwardly from the other of the flat surfaces of the base member, the stem member having a smaller cross-section than the base member and the axis of the stem member being off-set with respect to the center of and substantially non-perpendicular to the base surface of the base member.

Of the bio-active and bio-compatible glass and glass-ceramic compositions known to be useful for forming prostheses or surgical implant articles which bond to hard bone tissue, only a select few demonstrate the soft-tissue response necessary to enable their use to form prostheses for the middle ear. Most of the known bio-active, bio-compatible glasses require the imposition of a bone pate between the device fabricated therefrom and the soft tissue of the tympanic membrane to prevent extrusion therethrough and to otherwise stabilize the device within the middle ear.

The bacteriostatic, bio-compatible glass composition used to form the prosthesis of the present invention is preferably such that it:

(1) forms a strong adherent bond comprising a thin layer of collagen at a glass/hard bone tissue interface upon implantation in the middle ear;

(2) forms a strong, adherent bond comprising a thin layer of collagen no more than about 1 to 3 fibers thick at a glass/soft tissue interface upon implantation in the middle ear;

(3) becomes encapsulated after implantation in the middle ear with a thin collagen layer no more than about 1 to 3 collagen fibers thick;

(4) regenerates respiratory epithelium on the collagen encapsulated surface of (3);

(5) does not result in extrusion of the surgical implant article through the tympanic membrane; and

(6) does not result after implantation in the middle ear in the formation of scar tissue, giant cells or acute imflammatory cells.

Detailed description of the invention

In the preferred embodiments of the invention, the end of the stem member proximal to the base member is flared toward the base surface thereof.

In the drawings:

Figs. 1, 2, 3 and 4 are top plan views of various embodiments of the Middle Ear Prosthesis;

Figs. 1a, 2a, 3a and 4a are side elevational views of various embodiments of the Middle Ear Prosthesis, it being understood that the other sides correspond as in mirror images;

Figs. 1b, 2b, 3b and 4b are bottom plan views of various embodiments of the Middle Ear Prosthesis.

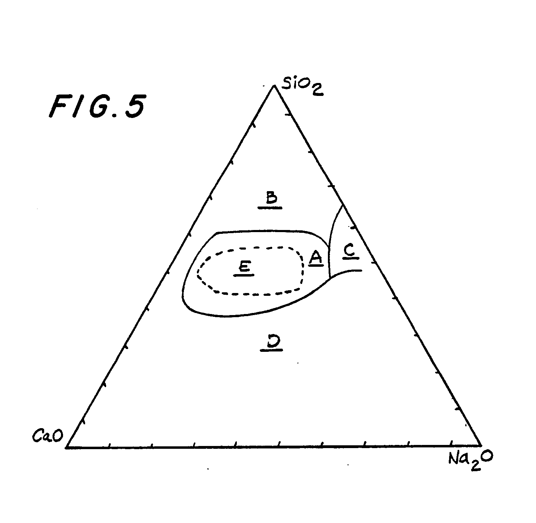

Fig. 5 is a compositional boundary design of $SiO_2$-$CaO$-$Na_2O$ glasses. Region A bounds those compositions which are bone-bonding glass formers. Region B comprises those compositions which are neither bone- or soft-tissue bonding glass formers. Region C defines those compositions which form glasses that dissolve in vivo when implanted in the body. Region D defines bone-bonding non-glass formers. Region E defines the compositions within region A which form glasses capable of bonding to bone and forming acceptable thin acellular collagen bonds with soft tissue in the middle ear.

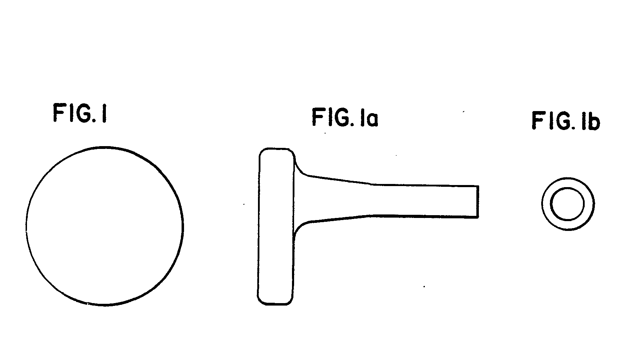

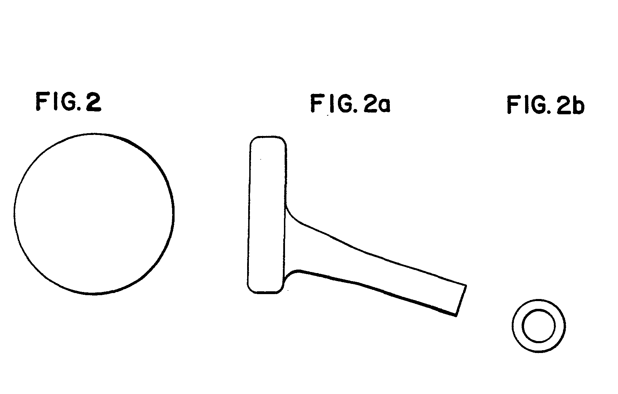

The middle ear prosthesis may take four different forms or shapes, each of which is specifically created to meet a specific anticipated surgical need. Two types of device (Figs. 1 and 2) have circular cross-section bases which are designed to interface with and bond to the thin collagen layer of the tympanic membrane or grafted tympanic membrane. The stem of the device, which is an integral part of the component, is designed to bridge the space of the middle ear and bond through a bio-active bond to the stapes footplate, stapes superstructures, incus or grafted membrane of the oval window.

The straight stem design (Figs. 1, 1a and 1b) is specifically for the surgical situation where no malleus handle and no stapes superstructure are present and the prosthesis stem must traverse the narrow oval window niche, but where the facial ridge is sufficiently high that the tympanic membrane or tympanic membrane graft is parallel to the stapes footplate or oval window graft membrane.

The angled stem (Figs. 2, 2a and 2b) is designed for the surgical situation where no stapes superstructure is present and the prosthesis stem must traverse the narrow oval window niche but where the facial ridge is sufficiently low that the tympanic membrane or tympanic membrane graft is angulated laterally from posterior to anterior, relative to the stapes footplate or oval window graft membrane.

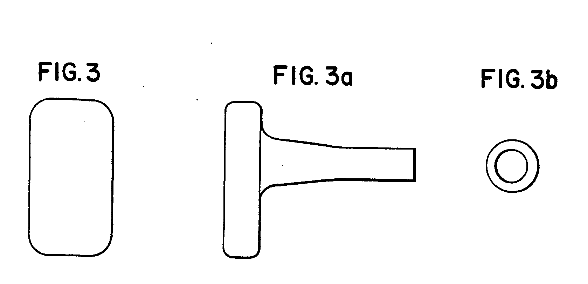

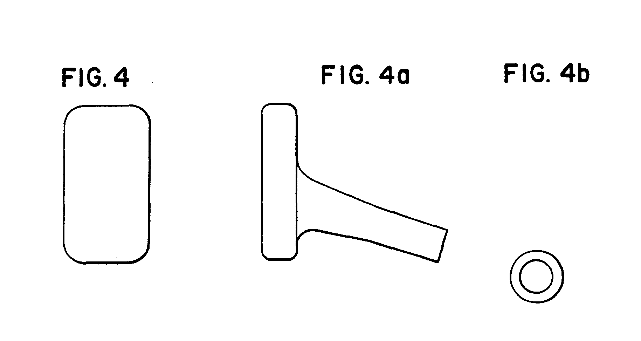

Two devices (Figs. 3 and 4) have generally rectangular bases which are designed to fit in contact with a malleus handle when the handle is present and to bond to the malleus handle and tympanic membrane or tympanic membrane graft.

The straight stem design (Figs. 3, 3a and 3b) is specifically for the surgical situation where the measured distance from head of stapes to malleus handle is within 0.1 inch (2.54 mm) in the anterior to posterior dimension.

The angled stem (see Figs. 4, 4a and 4b) is specifically for the surgical situation where the malleus handle is further than 0.1 inch (2.54 mm) in the anterior to posterior dimension and when a laterally displaced promontory does not allow clearance under the malleus handle.

In all designs the stem of the implant is specifically designed to have a relatively broad stem within 0.0945 inches (0.2400 mm) of the base then narrowing to 0.039 inches (0.991 mm) in diameter for the remaining distal portion of the stem. This narrow portion of ths stem will allow the stem to reside on the stapes footplate or oval window graft membrane with sufficient clearance of the walls of the oval window niche to avoid contact which might impede vibratory motion of the implant. The broader diameter stem nearer the base allows greater cross-sectional area for creating a facet for the head of the stapes to provide further mechanical stability to the implant on the stapes.

Currently available materials for use in middle ear sound transformer reconstruction include autograft and homograft bone and cartilage, Plasti-Pore® Proplast® and Ceraviral®. Autograft and homograft bone and cartilage require harvesting from human beings and in their natural shape are poorly designed for purposes of reconstruction and must be extensively machined prior to use. Plasti-Pore® and Proplast® are porous materials, the first of which is hard polyethylene and the second a relatively soft felt-like material. Neither material lends itself to micro-contouring to fit individual patient needs. Both of these materials require cartilage to be interposed between the implant and the tymponic membrane (graft) to avoid extrusion. Ceravital® is an opaque bio-active ceramic which requires the use of bone pate between the implant surface and soft tissue of the tympanic membrane (graft) to achieve a bond. In addition, Ceravital® is more difficult to contour than is the bio-active glass ear prosthesis of the invention.

Although a variety of glasses have been shown to bond to various soft tissue (Wilson et al, J. Biomed. Mater, Res., Vol *15*, pages 805 to 817), we have found that only a few of these glasses result in the formation of an exceptionally thin (i.e., not more than about 1—3 fibers thick) but adherent collagen film which strongly adheres the glass to the soft tissue without concomittant adverse side effects, thereby preserving the structural integrity of the middle ear.

These particular glass compositions have also been found to advantageously become encapsulated with a thin (i.e., no more than about 1—3 fibers thick) layer of collagen after implantation which allows regeneration of respiratory epithelium over the surface thereof thereby almost completely restoring the natural structure of the middle ear.

Generally, it has been found that bio-active and bio-compatible glasses having the following weight percent compositions give satisfactory results when utilized as materials of construction for total ossicular replacement prosthesis (TORP) or partial ossicular replacement prosthesis (PORP).

| Component | Weight percentage |
|-----------|-------------------|
| $SiO_2$ | 40—52 |
| CaO | 20—50 |
| $Na_2O$ | 10—35 |
| $P_2O_6$ | 2—8 |
| $CaF_2$ | 0—25 |
| $B_2O_3$ | 0—10 |

The following compositions have been found to yield optimum results and are, therefore, preferred:

| Component | Weight percentage |
|-----------|-------------------|
| $SiO_2$ | 45.0 |
| CaO | 24.5 |
| $Na_2O$ | 24.5 |
| $P_2O_5$ | 6.0 |

| Component | Weight percentage |
|-----------|-------------------|
| $SiO_2$ | 45 |
| CaO | 12.25 |
| $CaP_2$ | 12.25 |
| $P_2O_5$ | 6.0 |

| Component | Weight percentage |
|-----------|-------------------|
| $SiO_2$ | 40 |
| CaO | 24.5 |
| $Na_2O$ | 24.5 |
| $P_2O_5$ | 6.0 |
| $B_2O_3$ | 5.0 |

| Component | Weight percentage |
|-----------|-------------------|
| $SiO_2$ | 52 |
| CaO | 21.0 |
| $Na_2O$ | 21.0 |
| $P_2O_5$ | 6.0 |

The positioning of the bio-active glass middle ear prostheses of the invention at reconstruction can be confirmed as they are transparent as are none of the other available prostheses and can be contoured as easily as bone to create an implant with features specific to the needs of the individual patient. The bio-active glass middle ear prostheses of the invention can achieve stabilization within a reconstructed ossicular chain both through mechanical interlock provided by grooves and facets created at surgery into which other remaining parts of the ossicular chain are fitted and through bone and/or soft tissue bonding to remaining parts of the middle ear sound transformer system which may include any or all of the following tympanic membrane, tympanic membrane graft, malleus, incus, stapes superstructure, stapes footplate or oval window graft membrane.

All currently available middle ear prostheses have the general appearance of a tack or a stem and base but none combine the special characteristics of transparency, contourability and ability to bond to soft tissue and bone with design features with specific surgical reconstructive application as in the prostheses of the invention.

There are several advantages associated with the bio-active glass prostheses of the invention:

(1) the implants bond by means of a soft tissue bond to the tympanic membrane and to the remaining components of the ossicular chain.

(2) they have the advantage of ease of machining and ability to be contoured to meet the specification of the surgeon.

(3) they are transparent and therefore can be used with an optical microscope to visualize relationship in the ossicular chain in which they will be placed, and ensure proper positioning and contact at the time of surgery.

PORP or TORP prostheses may be formed from the bio-active, bio-compatible glass compositions by injection molding of the molten glass. In this method ths glass melt is raised to a temperature sufficient to give rise to fluidity under pressure (a range of 1300—1400°C) while enclosed to prevent evaporation of $Na_2O$ which is necessary to produce sufficient bio-activity to form the thin collagen bond. The molten glass is then poured into the spur of a mold (graphite, stainless steel, or boron nitride) previously heated to a temperature (about 300—500°C) to avoid solidification of the liquid to a glass prior to full adaptation to the mold. A positive or negative (vacuum) pressure is than rapidly applied to the mold via a small aperture. The molten liquid penetrates the mold forming the PORP or TORP and solidifies into a glass while doing so. The bio-active glass TORP or PORP is then removed from the mold and the casting sprue and pressure inlet is removed with a SiC cutting wheel and polished. Other shapes for middle ear devices can be made in an equivalent manner by varying the shape of the mold. Middle ear devices such as TORP's or PORP's can also be made in a two-step glass-bonding process. In this method, continuous fibers of a diameter suitable for the device (1.0—1.8 mm) are drawn from a glass melt and covered to prevent loss of alkali from the glass. The fibers are cut to appropriate lengths (2.0—8 mm) with a SiC wafering saw. The fibers are then inserted in the hole in a mold containing a cavity sufficient to form the head of the device. A drop of molten glass is then placed in the mold, thereby forming the head of the device and forming a bond with the fiber at the same time. The device is then easily removed from the mold.

## Claims

1. A middle ear prosthesis comprising a substantially disc-like base member, one base surface of which is especially adapted to interface with and bond to the thin collagen layer of the tympanic membrane or grafted tympanic membrane of the middle ear and an integral substantially cylindrical stem member projecting outwardly from the other base surface of said base member, said stem member having a smaller diameter than said base member and the axis of said stem member being off-set with respect to the center of and substantially perpendicular to said base surface, and wherein the prosthesis is constructed of bacteriostatic, bio-active and bio-compatible glass having the following weight percentage composition:

| Component | Weight percentage |
|-----------|-------------------|
| $SiO_2$ | 40—52 |
| CaO | 20—50 |
| $Na_2O$ | 10—35 |
| $P_2O_5$ | 2—8 |
| $CaF_2$ | 0—25 |
| $B_2O_3$ | 0—10 |

2. The middle ear prosthesis of claim 1, wherein the end of said stem member proximal to said base is flared.

3. A middle ear prosthesis comprising a substantially disc-like base member, one base surface of which is especially adapted to interface with and bond to the thin collagen layer of the tympanic membrane or grafted tympanic membrane of the middle ear and an integral substantially cylindrical stem member projecting outwardly from the other base surface of said base member, said stem member having a smaller diameter than said base member and the axis of said stem member being off-set with respect to the center of and substantially non-perpendicular to said base surface, and wherein the prosthesis is constructed of bacteriostatic, bio-active and bio-compatible glass having the following weight percentage composition:

| Component | Weight percentage |
|-----------|-------------------|
| $SiO_2$ | 40—52 |
| CaO | 20—50 |
| $Na_2O$ | 10—35 |
| $P_2O_5$ | 2—8 |
| $CaF_2$ | 0—25 |
| $B_2O_3$ | 0—10 |

4. The middle ear prosthesis of claim 3, wherein the end of said stem member proximal to said base is flared.

5. A middle ear prosthesis comprising a base member having two opposed flat surfaces, one of said flat surfaces being especially adapted to interface with and bond to a malleus handle of the middle ear and an integral substantially cylindrical stem member projecting outwardly from the other of said flat surfaces of said base member, said stem member having a smaller cross-section than said base member and the axis of said stem member being off-set with respect to the center of and substantially perpendicular to said base surface, and wherein the prosthesis is constructed of bateriostatic, bio-active and bio-compatible glass having the following weight percentage composition:

| Component | Weight percentage |
|-----------|-------------------|
| $SiO_2$ | 40—52 |
| CaO | 20—50 |
| $Na_2O$ | 10—35 |
| $P_2O_5$ | 2—8 |
| $CaF_2$ | 0—25 |
| $B_2O_3$ | 0—10 |

6. The middle ear prosthesis of claim 5 wherein the end of said stem member proximal to said base is flared.

7. A middle ear prosthesis comprising a base member having two opposed flat surfaces, one of said flat surfaces being especially adapted to interface with and bond to a malleus handle of the middle ear and an integral substantially cylindrical stem member projecting outwardly from the other of said flat surfaces of said base member, said stem member having a smaller cross-section than said base member and the axis of said stem member being off-set with respect to the center of and substantially non-perpendicular to said base surface, and wherein the prosthesis is constructed of bacteriostatic, bio-active and bio-compatible glass having the following weight percentage composition:

| Component | Weight percentage |
|-----------|-------------------|
| $SiO_2$ | 40—52 |
| CaO | 20—50 |
| $Na_2O$ | 10—35 |
| $P_2O_5$ | 2—8 |
| $CaF_2$ | 0—25 |
| $B_2O_3$ | 0—10 |

8. The middle ear prosthesis of claim 7 wherein the end of said stem member proximal to said base is flared.

9. A middle ear prosthesis as claimed in any one of claims 1 to 8, wherein the glass composition is substantially one of the following:

| Component | Weight percentage |
|-----------|-------------------|
| $SiO_2$ | 45 |
| CaO | 24.5 |
| $Na_2O$ | 24.5 |
| $P_2O_5$ | 6.0 |

| Component | Weight percentage |
|-----------|-------------------|
| $SiO_2$ | 45 |
| $CaO$ | 12.25 |
| $CaP_2$ | 12.25 |
| $P_2O_5$ | 6.0 |

| Component | Weight percentage |
|-----------|-------------------|
| $SiO_2$ | 40 |
| $CaO$ | 24.5 |
| $Na_2O$ | 24.5 |
| $P_2O_5$ | 6.0 |
| $B_2O_3$ | 5.0 |

| Component | Weight percentage |
|-----------|-------------------|
| $SiO_2$ | 52 |
| $CaO$ | 21.0 |
| $Na_2O$ | 21.0 |
| $P_2O_5$ | 6.0 |

**Patentansprüche**

1. Mittelohrprothese, die einen im wesentlichen scheibenförmigen Grundkörper umfaßt, dessen eine Basisfläche besonders angepaßt ist, um die Schnittstelle zu bilden und sich zu verbinden mit den dünnen Collagen-Lagen des Trommelfelles (typpanische Membran) oder eines transplantierten Trommelfelles des Mittelohres, und ein integriertes im wesentlichen zylindrisches Stielteil, das von der anderen Basisfläche des genannten Grundkörpers nach außen vorspringt, wobei das Stielteil einen kleineren Durchmesser hat als der Grundkörper und die Achse des Stielteiles versetzt ist in Bezug auf das Zentrum der Basisfläche des Grundkörpers und im wesentlichen senkrecht auf ihr steht, wobei die Prothese aus bakteriostatischem, bio-aktivem und bio-compatiblem Glas konstruiert ist, das die folgende prozentuale Gewichtszusammensetzung hat:

| Komponente | Prozentualer Gewichtsanteil |
|------------|------------------------------|
| $SiO_2$ | 40—52 |
| $CaO$ | 20—50 |
| $Na_2O$ | 10—35 |
| $P_2O_5$ | 2—8 |
| $CaF_2$ | 0—25 |
| $B_2O_3$ | 0—10 |

2. Mittelohrprothese gemäß Anspruch 1, wobei das Ende des genannten Stielteils nächst dem Fuß verdickt ist.

3. Mittelohrprothese, die einen im wesentlichen scheibenförmigen Grundkörper umfaßt, dessen eine Basisfläche besonders angepaßt ist, um die Schnittstelle zu bilden und sich zu verbinden mit den dünnen Collagen-Lagen des Trommelfelles (tympanische Membran) oder eines transplantierten Trommelfelles des Mittelohres, und ein integriertes im wesentlichen zylindrisches Stielteil, das von der anderen Basisfläche des genannten Grundkörpers nach außen vorspringt, wobei das Stielteil einen kleineren Durchmesser hat als der Grundkörper und die Achse des Stielteiles versetzt ist in Bezug auf das Zentrum der Basisfläche des Grundkörpers und im wesentlichen nicht senkrecht auf ihr steht, wobei die Prothese aus bakteriostatischem, bio-aktivem und bio-composiblem Glas konstruiert ist, das die folgende prozentuale Gewichtszusammensetzung hat:

| Komponente | Prozentualer Gewichtsanteil |
|------------|------------------------------|
| $SiO_2$ | 40—52 |
| $CaO$ | 20—50 |
| $Na_2O$ | 10—35 |
| $P_2O_5$ | 2—8 |
| $CaF_2$ | 0—25 |
| $B_2O_3$ | 0—10 |

4. Mittelohrprothese gemäß Anspruch 3, wobei das Ende des genannten Stielteils nächst dem Fuß verdickt ist.

5. Mittelohrprothese, die einen Grundkörper mit zwei gegenüberliegenden flachen Oberflächen enthält, wobei eine dieser Oberflächen besonders angepaßt ist, um die Schnittstelle zu bilden und sich zu verbinden mit dem Hammergriff des Mittelohres und einem integrierten im wesentlichen zylindrischen Stielteil, das von der anderen der flachen Flächen des Grundkörpers nach außen vorspringt, wobei das Stielteil einen kleineren Querschnitt hat als der Grundkörper und die Achse des Stielteiles versetzt ist in Bezug auf das Zentrum der Basisfläche des Grundkörpers und im wesentlichen senkrecht auf ihr steht, wobei die Prothese aus bakteriostatischem, bio-aktivem und bio-compatiblem Glas konstruiert ist, das die folgende prozentuale Gewichtszusammensetzung hat:

| Komponente | Prozentualer Gewichtsanteil |
|------------|------------------------------|
| $SiO_2$ | 40—52 |
| $CaO$ | 20—50 |
| $Na_2O$ | 10—35 |
| $P_2O_5$ | 2—8 |
| $CaF_2$ | 0—25 |
| $B_2O_3$ | 0—10 |

6. Mittelohrprothese gemäß Anspruch 5, wobei das Ende des genannten Stielteils nächst dem Fuß verdickt ist.

7. Mittelohrprothese, die einen Grundkörper mit zwei gegenüberliegenden flachen Oberflächen enthält, wobei eine dieser Oberflächen besonders angepaßt ist, um die Schnittstelle zu bilden und sich zu verbinden mit dem Hammergriff des Mittelohres und einem integrierten im wesentlichen zylindrischen Stielteil, das von der anderen der flachen Flächen des Grundkörpers nach außen vorspringt, wobei das Stielteil einen kleineren Querschnitt hat als der Grundkörper und die Achse des Stielteiles versetzt ist in Bezug auf das Zentrum der Basisfläche des Grundkörpers und im wesentlichen nicht senkrecht auf ihr steht, wobei die Prothese aus bakteriostatischem, bio-aktivem und bio-compatiblem Glas konstruiert ist, das die folgende prozentuale Gewichtszusammensetzung hat:

| Komponente | Prozentualer Gewichtsanteil |
|---|---|
| $SiO_2$ | 40—52 |
| CaO | 20—50 |
| $Na_2O$ | 10—35 |
| $P_2O_5$ | 2—8 |
| $CaF_2$ | 0—25 |
| $B_2O_3$ | 0—10 |

8. Mittelohrprothese gemäß Anspruch 7, wobei das Ende des genannten Stielteils nächst dem Fuß verdickt ist.

9. Mittelohrprothese nach einem der vorhergehenden Ansprüche 1 bis 8, wobei die Glaszusammensetzung im wesentlichen eine der folgenden ist:

| Komponente | Prozentualer Gewichtsanteil |
|---|---|
| $SiO_2$ | 45 |
| CaO | 24.5 |
| $Na_2O$ | 24.5 |
| $P_2O_5$ | 6.0 |

| Komponente | Prozentualer Gewichtsanteil |
|---|---|
| $SiO_2$ | 45 |
| CaO | 12.25 |
| $CaP_2$ | 12.25 |
| $P_2O_5$ | 6.0 |

| Komponente | Prozentualer Gewichtsanteil |
|---|---|
| $SiO_2$ | 40 |
| CaO | 24.5 |
| $Na_2O$ | 24.5 |
| $P_2O_5$ | 6.0 |
| $B_2O_3$ | 5.0 |

| Komponente | Prozentualer Gewichtsanteil |
|---|---|
| $SiO_2$ | 52 |
| CaO | 21.0 |
| $Na_2O$ | 21.0 |
| $P_2O_5$ | 6.0 |

**Revendications**

1. Prothèse pour l'oreille moyenne comprenant un élément de base sensiblement en forme de disque, une surface de base qui est particulièrement adaptée à servir d'interface à la mince couche de collagène de la membrane tympanique ou à la membrane tympanique greffés de l'oreille moyenne et à se lier avec elle et un élément en forme de tige sensiblement cylindrique d'un seul tenant se projetant vers l'extérieur à partir de l'autre surface de base dudit élément de base, ledit élément formant tige ayant un diamètre plus petit que celui dudit élément de base et l'axe dudit élément formant tige étant décalé par rapport au centre de ladite surface de base et sensiblement perpendiculaire à celle-ci, ladite prothèse étant réalisée en un verre bactériostatique, bioactif et biocompatible ayant la composition suivante exprimée en pourcentage pondéral:

| Composant | Pourcentage pondéral |
|---|---|
| $SiO_2$ | 40—52 |
| CaO | 20—50 |
| $Na_2O$ | 10—35 |
| $P_2O_5$ | 2—8 |
| $CaF_2$ | 0—25 |
| $B_2O_3$ | 0—10 |

2. Prothèse pour l'oreille moyenne selon la revendication 1, dans laquelle l'extrémité proximale, par rapport à ladite base, dudit élément formant tige est évasée.

3. Prothèse pour l'oreille moyenne comprenant un élément de base sensiblement on forme de disque, une surface de base qui est particulièrement adaptée à service d'interface à la mince couche de collagène de la membrane tympanique ou à la membrane tympanique greffée de l'oreille moyenne, et à se lier avec elle, et un élément en forme de tige sensiblement cylindrique d'un seul tenant se projetant vers l'extérieur à partir de l'autre surface de base dudit élément de base, ledit élément formant tige ayant un diamètre plus petit que celui dudit élément de base et l'axe dudit élément formant tige étant décalé par rapport au centre de ladite surface de base et sensiblement non-perpendiculaire à celle-ci, ladite prothèse étant réalisée en un verre bactériostatique, bioactif et biocompatible ayant la composition suivante exprimée en pourcentage pondéral:

| Composant | Pourcentage pondéral |
|---|---|
| $SiO_2$ | 40—52 |
| CaO | 20—50 |
| $Na_2O$ | 10—35 |
| $P_2O_5$ | 2—8 |
| $CaF_2$ | 0—25 |
| $B_2O_3$ | 0—10 |

4. Prothèse pour l'oreille moyenne selon la revendication 3, dans laquelle l'extrémité proximale, par rapport à ladite base, dudit élément formant tige est évasée.

5. Prothèse pour l'orielle moyenne comprenant un élément de base ayant deux surfaces plates opposées, l'une desdites surfaces plates étant particulièrement adaptée à servir d'interface à un manche de marteau de l'oreille moyenne, et à se lier avec lui, et un élément formant tige sensiblement cylindrique d'un seul tenant se projetant vers l'extérieur à partir de l'autre desdites surfaces plates dudit élément de base, ledit élément formant tige ayant une section transversale plus petite que celle de l'élément de base et l'axe dudit élément formant tige étant décalé par rapport au centre de ladite surface de base et sensiblement perpendiculaire à celle-ci, la prothèse étant réalisée en un verre bactériostatique, bioactif et biocompatible ayant la composition suivante exprimée en pourcentage pondéral:

| Composant | Pourcentage pondéral |
|-----------|---------------------|
| $SiO_2$ | 40—52 |
| CaO | 20—50 |
| $Na_2O$ | 10—35 |
| $P_2O_5$ | 2—8 |
| $CaF_2$ | 0—25 |
| $B_2O_3$ | 0—10 |

6. Prothèse pour l'oreille moyenne selon la revendication 5, dans laquelle l'extrémité proximale, par rapport à ladite base, dudit élément formant tige est évasée.

7. Prothèse pour oreille moyenne comprenant un élément de base ayant deux surfaces plates opposées, l'une desdites surfaces plates étant particulièrement adaptée à servir d'interface avec un manche de marteau de l'oreille moyenne, et à se lier avec lui, et un élément formant tige sensiblement cylindrique d'un seul tenant se projetant vers l'extérieur à partir de l'autre desdites surfaces plates dudit élément de base, ledit élément formant tige ayant une section transversale plus petite que celle de l'élément de base et l'axe dudit élément formant tige étant décalé par rapport au centre de ladite surface de base et sensiblement non-perpendiculaire à celle-ci, la prothèse étant réalisée en un verre bactériostatique, bioactif et biocompatible ayant la composition suivante exprimée en pourcentale pondéral:

| Composant | Pourcentage pondéral |
|-----------|---------------------|
| $SiO_2$ | 40—52 |
| CaO | 20—50 |
| $Na_2O$ | 10—35 |
| $P_2O_5$ | 2—8 |
| $CaF_2$ | 0—25 |
| $B_2O_3$ | 0—10 |

8. Prothèse pour l'oreille moyenne selon la revendication 7, dans laquelle l'extrémité proximale, par rapport à ladite base, dudit élément formant tige est évasée.

9. Prothèse pour l'oreille moyenne selon l'une quelconque des revendications 1 à 8, dans laquelle la composition de verre répond sensiblement à l'une des suivantes:

| Composant | Pourcentage pondéral |
|-----------|---------------------|
| $SiO_2$ | 45 |
| CaO | 24,5 |
| $Na_2O$ | 24,5 |
| $P_2O_5$ | 6,0 |

| Composant | Pourcentage pondéral |
|-----------|---------------------|
| $SiO_2$ | 45 |
| CaO | 12,25 |
| $CaP_2$ | 12,25 |
| $P_2O_5$ | 6,0 |

| Composant | Pourcentage pondéral |
|-----------|---------------------|
| $SiO_2$ | 40 |
| CaO | 24,5 |
| $Na_2O$ | 24,5 |
| $P_2O_5$ | 6,0 |
| $B_2O_3$ | 5,0 |

| Composant | Pourcentage pondéral |
|-----------|---------------------|
| $SiO_2$ | 52 |
| CaO | 21,0 |
| $Na_2O$ | 21,0 |
| $P_2O_5$ | 6,0 |

FIG. I

FIG. Ia

FIG. Ib

FIG. 2            FIG. 2a            FIG. 2b

FIG. 3          FIG. 3a          FIG. 3b

FIG. 4          FIG. 4a          FIG. 4b

FIG.5